# EUROPEAN PATENT SPECIFICATION

(11) **EP 3 569 595 B1**
(45) Date of publication and mention of the grant of the patent: **30.06.2021**
(21) Application number: 19174557.9
(22) Date of filing: 15.05.2019
(51) Int. Cl.: C07D 251/62, B01D 43/00, B01D 21/26, B04B 1/00

(54) **DEVICE FOR SEPARATING MELAMINE CRYSTALS IN A PRODUCTION PROCESS OF MELAMINE FROM UREA**
VORRICHTUNG ZUM ABTRENNEN VON MELAMINKRISTALLEN IN EINEM HERSTELLUNGSPROZESS VON MELAMIN AUS HARNSTOFF
DISPOSITIF DE SÉPARATION DE CRISTAUX DE MÉLAMINE DANS UN PROCÉDÉ DE PRODUCTION DE MÉLAMINE À PARTIR D'URÉE

(30) Priority: 15.05.2018 IT 201800005382
(43) Date of publication of application: 20.11.2019
(73) Proprietor: EUROTECNICA MELAMINE AG, 8832 Wollerau (CH)
(72) Inventor: SANTUCCI, Roberto, 21050 Gorla Maggiore (VA) (IT)
(74) Representative: De Gregori, Antonella

(56) References cited:
- IT-A1- MI20 061 589
- BELL GEORGE R A ET AL: "Mathematical model for solids transport power in a decanter centrifuge", CHEMICAL ENGINEERING SCIENCE, OXFORD, GB, vol. 107, 16 December 2013 (2013-12-16), pages 114-122, XP028856418, ISSN: 0009-2509, DOI: 10.1016/J.CES.2013.12.007

## Description

The present invention relates to a device for separating melamine crystals, i.e. melamine in the solid crystalline state, in a production process of melamine from urea. In most processes for the production of melamine from urea, the product leaving the synthesis (or pyrolysis) reactor is dissolved in water or in an aqueous solution recovered from downstream processing, then subjected to the specific purification treatment of the particular technology adopted and the purified melamine is finally separated from the mother liquor containing it by means of a crystallization process. Impurities, by-products and/or reaction intermediates leave the pyrolysis reactor together with the melamine. The end-product, i.e melamine, must be purified as much as possible, separating these impurities, by-products and/or reaction intermediates, and there are various technologies that use different methods, all of them directed towards this purpose. The separation of the crystallized melamine from the crystallization mother liquor can therefore be effected using different methods known to skilled persons in the field.

The production process of melamine from urea described in patent EP2385043B1 provides, for example, for the separation of purified melamine from an aqueous solution containing high quantities of ammonia. Said aqueous ammonia solution leaves the final purification step at a temperature and pressure ranging from 140 to 230°C and from 25 to 30 barg respectively, these conditions being necessary for maintaining both the ammonia, present at a concentration ranging from 8 to 28% by weight, and the melamine itself, in solution. Said aqueous ammonia solution containing in solution, all the melamine produced in the pyrolysis reactor of urea, is subjected according to patent EP2385043B1 to decompression and contemporaneous cooling in a crystallization apparatus at a pressure slightly higher than atmospheric pressure, ranging from 0.1 to 0.5 barg, and a temperature of 40-50°C, to separate most of the melamine contained therein in the crystalline solid state.

In this way, it is possible to obtain the recovery of most of the melamine in a state of high purity, whereas the ammonia that accompanies it remains practically all in aqueous solution, guaranteeing that the oxyaminotriazines (OATs), by-product of the synthesis reaction, remain in solution.

The aqueous ammonia solution coming from the final purification state mentioned above, can contain up to 23% by weight of melamine, in common industrial practice, however, the melamine content is maintained at between 6 and 15% by weight, more preferably between 10 and 15%.

As the quantity of residual melamine in the mother liquor, under the crystallization conditions, after separation of the melamine crystals precipitated, ranges from 0.8 to 1.1% by weight, the recovery rate of the melamine from the ammonia solution containing it can reach values ranging from 90 to 96% by weight.

As specified above, it should also be remembered that, in reality, the mother liquor contains not only melamine, but also by-products, such as for example oxyaminotriazines (OATs) which are kept in solution, up to certain concentrations, by the presence of ammonia which guarantees the necessary pH.

Oxyaminotriazines represent an impurity which pollutes the end-product and consequently their content must be minimized. For this reason, the mother liquor obtained after the separation of the crystallized melamine cannot be recycled as such, as this recycling would increase the concentration of OATs in the purification phase and, consequently, this increase in concentration would lead to the precipitation of the OATs together with the melamine in the crystallization phase.

The separation and purification step of melamine according to EP2385043B1 does not provide for the presence of a thickener, which, on the contrary, is provided in other systems of the prior art, such as, for example, US 3,161,638 in which it allows an increase in the size of the crystal, proceeding with a washing with ammonia water and removing the mother liquor from the slurry, thus reducing the content of oxyaminotriazines (OATs) in the solution containing the crystals.

The foregoing clearly shows that, during the separation of melamine from the mother liquor, it should be avoided that
- solid crystals of melamine remain in the mother liquor, as this melamine would be lost, consequently decreasing the yield of the process and plant;
- an excessive quantity of mother liquor remains in the melamine cake as, in the subsequent drying step, as the by-products contained in the mother liquor are not volatile, they would remain in the final product, i.e. in the melamine, decreasing its purity.

The separation of the crystallized melamine from the crystallization mother liquor can be carried out in different ways known to skilled persons in the field, i.e. by means of vacuum filtration, pressure filtration, continuous or discontinuous centrifugation, forced decantation.

In selecting the separation medium, a skilled person in the art should however bear in mind whether or not the mother liquor contains ammonia, as the presence of ammonia prevents the use of vacuum filters, both for environmental reasons (ammonia would be dispersed in the environment) and also for process reasons (the vacuum extracts the ammonia from the solution eliminating its effect).

A different, but more expensive, way is to separate the crystallized melamine from the slurry leaving the crystallizer and containing ammonia, using a pressure filter so as to operate safely despite the presence of ammonia.

The pressure filtration operation can be of the discontinuous or continuous type. Discontinuous pressure filtration requires a considerable use of labour for managing the loading and unloading cycle of the filter, with high dead times also due to the need for cleaning the environment during the discharge of the cake which is soaked with ammonia-saturated mother liquor. The handling of the cake, which must be sent to the subsequent drying operation, also has considerable problems due to the presence of ammonia.

Pressure filtration in continuous overcomes, in principle, all the health and safety problems present in discontinuous filtration. In reality, however, even the most perfected equipment for continuous filtration has problems from a mechanical point of view, which have not yet been satisfactorily solved, linked to the presence of extended creeping sealing surfaces that limit their operational continuity. This kind of equipment is in fact subjected to frequent stoppages due to mechanical problems, wear of the creeping seals and the overall delicacy of the structure.

Not even does the use of discontinuous centrifuges, on the other hand, guarantee the necessary running continuity unless a certain number of discontinuous centrifuges in parallel are used, with considerable labour effort. The use of various types of continuous centrifuges, for example those with a filtering basket of the Pusher type, overcomes environmental drawbacks and the problem of operating continuity, but specifically designed centrifuges must be used, based on the specific weight and, above all, the particle size of the product to be treated, in this case melamine, as otherwise standard continuous centrifuges are subject to yield variations with loss of melamine. There are, in fact, losses of melamine due to the melamine which exits with the mother liquor or as a result of the excessive quantity of mother liquor which remains in the product cake, thus jeopardizing the quality of the end-product and overcharging the drying section downstream.

The melamine crystals can then be separated by continuous centrifugation in a particular type of centrifuge without filtering parts, such as, for example, metal nets and/or canvases, but which acts as a decanter in which the decantation does not take place by gravity but by the effect of centrifugal force, thus obtaining a forced decantation. This separation method solves most of the problems indicated above, but it has been surprisingly found that only by effecting this method according to precise conditions in a device with particular characteristics, can a particularly pure product be obtained and with a further reduction in the melamine crystals remaining in suspension and which are therefore eliminated with the crystallization mother liquor. The present invention is therefore particularly advantageous when the synthesis process of melamine provides, in the crystallization phase, for maintaining by-products in solution in the presence of crystallized melamine, such as, for example, in the case of the synthesis process of melamine described in EP2385043B1, also characterized by the presence of quantities of crystals having reduced dimensions due to the absence of a thickener in the separation and purification system of melamine. This situation of maintaining by-products in solution occurs every time crystallization takes place in the presence of an alkaline agent which maintains the pH of the solution at values higher than 10, preferably higher than 11.5.

The objective of the present invention is therefore to overcome the above-mentioned drawbacks of the state of the art.

A process is disclosed for the recovery of melamine from the crystallization mother liquor which is particularly effective and safe. It is disclosed a process for separating melamine crystals in suspension (slurry) in the crystallization mother liquor characterized in that it comprises the following steps:
- a step for feeding a suspension comprising from 6 to 15% by weight of crystalline melamine with respect to the total weight of the suspension, with a quantity of crystals having a diameter smaller than 0.053 mm, lower than or equal to 40% by weight with respect to the total amount of melamine,
- a forced decantation step wherein a liquid phase and a solid phase are separated, the liquid phase comprising the mother liquor with an increase in total solids not exceeding 0.1% by weight with respect to the solid content in solution present in the mother liquor being fed, the solid phase consisting of a wet cake of melamine crystals with a residual content of mother liquor ranging from 5 to 18% by weight, more preferably from 7 to 13% by weight, even more preferably from 8 to 10% by weight with respect to the total weight of the cake.

The disclosed process for the separation of melamine crystals in suspension is particularly advantageous as it allows a liquid phase to be obtained, i.e. the mother liquor which is removed, wherein the total solids are present in a minimum quantity, not increasing more than 0.1% by weight with respect to the content of solids in solution already present in the mother liquor being fed (equal to about 1% by weight) and a content of mother liquor in the melamine cake which is less than 18%.

The mother liquor removed contains 1% by weight of melamine in solution, which cannot be recovered as it is dissolved, whereas the quantity of melamine remaining in suspension is, at the most, equal to about 0.1% by weight.

The process allows, starting from mother liquor having a melamine content of 10% by weight of which 1% is in solution and the remaining part in suspension, about 99% by weight of the melamine present in suspension to be recovered, i.e. about 89% by weight of the total melamine present in the mother liquor.

A further advantage lies in the fact that the solid phase, consisting of the wet cake of melamine crystals separated from the liquid phase (i.e. the mother liquor), contains a quantity of mother liquor lower than 18%, thus reducing the amount of possible by-products which pollute the purified end-product.

Furthermore, despite the high content of fine products, the crystals having a diameter of less than 0.053 mm can in fact constitute up to 40% by weight with respect to the total amount of melamine, the quantity of mother liquor remaining in the cake does not increase with time, contrary to what occurs in a filtration system, due to a progressive clogging of the filtering element.

Processes and devices for forced decantation according to the state of the art, which do not have the particular selection of characteristics of the present invention, also have a progressive clogging in the area of the centrifuge into which the water flows, thus causing a progressive increase in the content of mother liquor in the cake and after only a week of activity, a backwash of the centrifuge must be carried out. The backwash of the centrifuge requires the blockage of the same which must be washed with water and this also involves the need for storing the product to be fed to the centrifuge for the whole blocking time of the same. The solution according to the present invention, on the contrary, despite the high content of fine products, allows the backwash to be effected only once a month or even less frequently.

The term "forced" refers to a decantation effected according to specific conditions, characterized by the presence of a field of mass forces acting on the suspension to be separated, considerably above the gravitational force field. In particular, the process for separating melamine crystals according to the present invention provides a centrifugal decantation step conducted at the same crystallization temperature as the melamine (which in industrial practice ranges from 40 to 55°C), the slurry to be separated, consisting of melamine crystals in suspension in the crystallization mother liquor, being subjected to a suitably high centrifugal acceleration field, such as to facilitate the rapid decantation of the above-mentioned crystals, at the same time obtaining clear mother liquor.

In particular, it has been found that a centrifugal acceleration field within the range of 500 to 5,000r, preferably ranging from 1,500 to 2,500r, r being the acceleration of gravity equal to 9.81 meters per second per second, is adequate for obtaining a rapid and effective separation of the slurry into the two liquid and solid phases of which it is composed.

The forced decantation step of the recovery process according to the present invention is therefore carried out in the presence of a centrifugal acceleration field within the range of 500 to 5,000 r, preferably from 1,500 to 2,500 r.

The forced decantation step of the recovery process according to the present invention, separates in continuous the solid phase consisting of the mass of melamine crystals from the liquid phase (i.e. mother liquor) in the presence of an alkaline agent, at the same time ensuring the maintenance of a pH higher than 10, such as for example ammonia, more preferably higher than 11.5.

The solid phase consisting of the wet cake of melamine crystals is then fed mechanically and directly to the subsequent drying phase.

Object of the present invention relates to a device for implementing the process for separating melamine crystals in a plant for the synthesis of melamine from urea, said device being a full basket centrifuge consisting of a stationary external case in which a rotating basket is positioned, suitable for separating the two phases, solid and liquid, said rotating basket consisting of a hollow cylindrical body C terminating, on the outlet side of the wet cake, with a squeezing section S having a truncated-conical form, said truncated-conical squeezing section S being characterized by an angle α ranging from 6 to 16°, more preferably from 9 to 13°, said angle α being the inclination angle of the section S, measured with respect to the rotation plane, and said device being characterized in that the S'/C' ratio ranges from 0.45 to 0.85, preferably from 0.5 to 0.65, C' being the length of the cylindrical body and S' being the projection length of the truncated-conical squeezing section S on the rotation axis.

The squeezing section has the function of draining the liquid phase from the melamine crystal cake as much as possible, and the specific angulation of the truncated-conical form, as well as the ratio between the length of the cylindrical portion of the rotating basket and the projection length of the truncated-conical part, i.e. of the squeezing section on the rotation axis, surprisingly allow a particularly advantageous separation to be obtained, wherein the centrifuge of the Decanter or full basket type, receiving a suspension (slurry) comprising from 6 to 15% by weight of crystalline melamine with respect to the total weight of the suspension, with a quantity of crystals having a diameter of less than 0.053 mm, less than or equal to 40% by weight, with respect to the total quantity of melamine, separates a liquid phase and a solid phase, the liquid phase comprising the mother liquor with an increase in total solids not exceeding 0.1% by weight with respect to the solid content present in solution in the mother liquor being fed, the solid phase consisting of a wet cake of melamine crystals with a residual content of mother liquor ranging from 5 to 18% by weight, more preferably from 7 to 13% by weight, even more preferably from 8 to 10% by weight with respect to the total weight of the cake.

The full basket centrifuge makes it possible to operate in continuous and in complete isolation with the external working environment, feeding the wet cake mechanically and directly to the subsequent drying equipment, without any intervention of the operators.

Furthermore, the specific selection of the dimensions and ratios indicated above with respect to the squeezing section and the rotating basket, allows the device according to the present invention to avoid clogging of the water outflow area in the squeezing section and therefore to effect the backwash of the centrifuge once a month or even less, despite the high content of fine products, avoiding a progressive increase in the content of mother liquor in the cake which is kept within reduced limits.

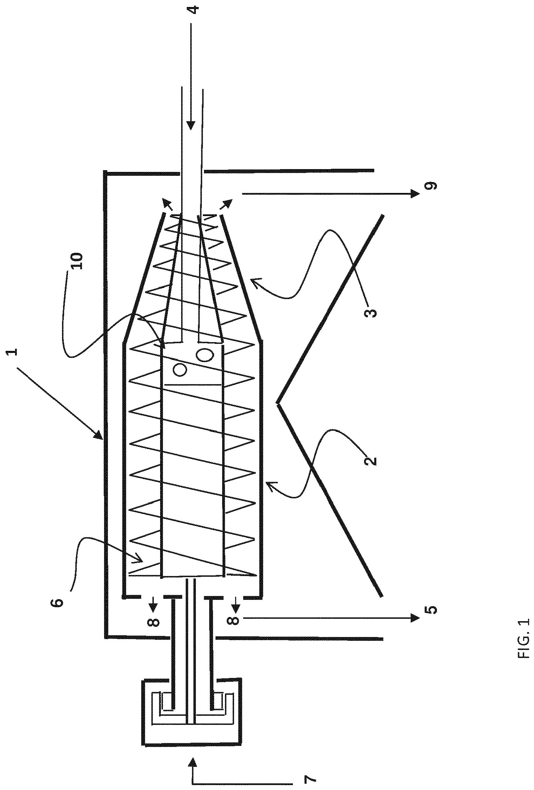

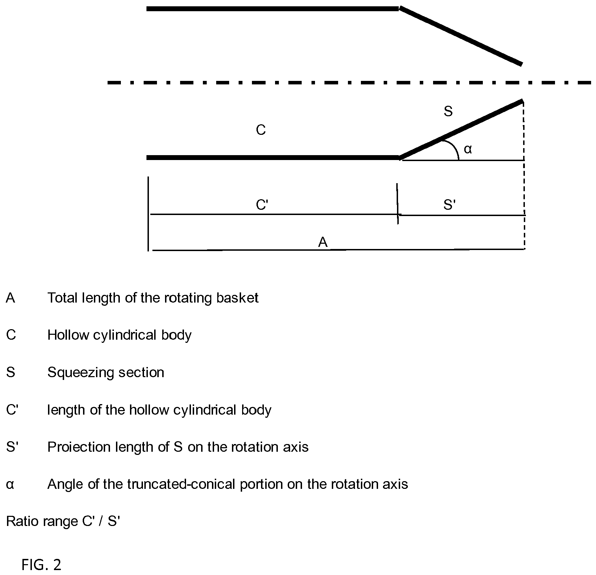

Without limiting the extension of the applicative solution claimed, an application embodiment of the device according to the present invention is exemplified in figure 1 which schematically represents the functioning principle of the device. Figure 2 schematically shows the elements of the rotating basket and the relative positioning of the cylindrical body and squeezing section.

The full basket centrifuge essentially consists of an external stationary case (1) in which a rotating basket (2) is positioned, which, thanks to its rotation, provides the centrifugal energy necessary for separating the two phases, solid and liquid and based on their different density.

The rotating basket (2), which is the rotating element, consists of a hollow cylindrical body terminating, on the outlet side of the wet cake, with a squeezing section (3) having the function of draining the liquid phase as much as possible from the cake of melamine crystals.

Said truncated-conical squeezing section S (3) has an angle α ranging from 6 to 16°, more preferably from 9 to 13°, said angle α being the inclination angle of the section S measured with respect to the rotation plane. Furthermore, the S'/C' ratio of the device according to the present invention ranges from 0.45 to 0.85, preferably from 0.5 to 0.65, wherein C' is the length of the cylindrical body and S' is the projection length of the truncated-conical squeezing section S on the rotation axis of the rotating basket, as shown in detail in figure 2.

The aqueous ammonia slurry leaving the crystallizer is fed into the rotating basket (2) of the centrifuge by means of the slurry feeding tube (4). The inlet position (10) of the aqueous ammonia slurry into the rotating basket (2) affects the efficiency of the solid/liquid separation.

If the feeding of the slurry (10) is positioned too close to the outlet of the solid phase, there will in fact be too much residual water in the wet cake leaving the centrifuge, whereas, viceversa, if the feeding of the slurry (10) is positioned too close to the outlet of the liquid phase, there will be crystals in suspension in the liquid phase leaving the outlet nozzle of the clarified liquid (5).

The preferred position, as it is more effective, of the entry point of the slurry fed or inlet chamber of the slurry (10), lies in correspondence with the connection point between the cylindrical portion and the truncated-conical portion of the rotating basket, which corresponds to the beginning of the squeezing section. By feeding the slurry in this point, in fact, the efficiency of the ratio between the cylindrical part and the truncated-conical part is fully exploited.

Due to the centrifugal acceleration imparted by the rotation of the rotating basket (2), the melamine crystals, having a density higher than the liquid in which they are suspended, thicken and are deposited against the wall of the rotating basket (2). Inside the rotating basket (2), the rotating mass is stratified in a solid phase which is arranged in the outer circumferential part and in a liquid phase, free of suspended crystals (maximum quantity about 0.1% by weight), which settles above the solid phase.

The movement of the solid phase towards the outlet is obtained by means of the extraction screw (6) whose differential motion with respect to the rotating basket (2) is guaranteed by the reducer (7). Said rotating screw (6) is a double volute screw, i.e. having two starting points: the height of the solid transported by each coil is thus halved, facilitating the outflow of the liquid phase.

In particular, it has been surprisingly found that the best reduction ratio between rotating basket and screw ranges from 95:1 to 70:1, more preferably from 90:1 to 77:1. The liquid phase leaves the rotating basket (2) by means of specific adjustable openings (8), whose position determines the height of the liquid phase inside the rotating basket (2) and consequently the useful length, i.e. not covered by the liquid phase, of the squeezing section (3), with the same angle α and the same the S'/C' ratio between the length of the cylindrical portion C' and the projection length of the truncated-conical portion on the rotation axis of the rotating basket S'.

The specific selection of the dimensions and ratios indicated above have surprisingly led to obtaining improved performances in the solid/liquid separation with a maximum increase in the total solids contained in the mother liquor of 0.1% by weight with respect to the content of solids in solution present in the mother-liquor being fed and with a solid phase consisting of a wet cake of melamine crystals with a residual content of mother liquor ranging from 5 to 18% by weight, more preferably from 7 to 13% by weight, even more preferably from 8 to 10% by weight with respect to the total weight of the cake.

At the opposite sides of the rotating basket, there is the outlet of the clarified mother liquor (5) and the outlet of the wet melamine cake (9).

The advantages of using a centrifuge decanter in the process for separating melamine crystals from the crystallization slurry are listed hereunder:
i. the separation process of the solid phase from the liquid phase is continuous and takes place under conditions of complete isolation from the surrounding working environment. No leakage of ammonia is possible during the separation and transfer operation of the wet cake downstream;
ii. the solid is effectively separated from the mother liquor that continuously exits from the equipment perfectly clear;
iii. the solid is effectively drained from the liquid phase in the squeezing section (3) (see figure 1). The residual content of mother liquor in the solid discharged preferably ranges from 8 to 10% by weight and is in any case less than 18% by weight. The discharge of the solid also takes place in continuous, without any contact with the surrounding working environment, therefore under high safety conditions;
iv. the seals between the rotating parts and the fixed parts of the centrifuge are of standard construction and ensure a long period of operation without any maintenance and/or inconveniences;
v. maximum safety for the operators as no manual operations are foreseen and as it is impossible for the operators themselves to reach moving parts;
vi. the specific selection of the dimensions and ratios indicated for the squeezing section and the rotating basket, allows the device of the present invention to avoid clogging of the water outflow area in the squeezing section thus enabling the backwash of the centrifuge to be effected once a month or even less, despite the high content of fine products, avoiding a progressive increase in the content of mother liquor in the cake which is always kept within reduced limits.

The equipment ensures an annual operating factor of over 8,000 hours, helping to ensure maximum productivity of the entire plant.

## Claims

1. A device for implementing a process for the separation of melamine crystals in a plant for the synthesis of melamine from urea, said device being a full basket centrifuge, consisting of a stationary external case (1) in which a rotating basket (2) is positioned, suitable for separating the two phases, solid and liquid, said rotating basket (2) consisting of a hollow cylindrical body C terminating, on the outlet side of the wet cake, with a squeezing section S (3) having a truncated-conical form, said truncated-conical squeezing section S (3) being **characterized by** an angle α ranging from 6 to 16°, said angle α being the inclination angle of said section S, measured with respect to the rotation plane and said device being **characterized in that** the S'/C' ratio ranges from 0.45 to 0.85, C' being the length of the cylindrical body and S' being the projection length of the truncated-conical squeezing section (S) on the rotation axis; said device being in a plant for the synthesis of melamine from urea.

2. The device according to claim 1, **characterized in that** the centrifuge comprises an extraction screw (6), whose differential motion with respect to the rotating basket (2) is ensured by the reducer (7).

3. The device according to claim 2, **characterized in that** the extraction screw (6) is a double volute screw.

4. The device according to one or more of claims 1-3, **characterized in that** the rotating basket (2) has adjustable openings (8), whose position determines the height of the liquid phase inside the rotating basket (2) and consequently the useful length, not covered by the liquid phase, of the squeezing section (3), with the same angle α and S'/C' ratio between the length of the cylindrical portion and the projection length of the truncated-conical portion on the rotation axis of the rotating basket.

5. The device according to one or more of claims 1-4, **characterized in that** the position (10) of the inlet point (4) of the slurry fed corresponds to the connection point between the cylindrical portion and the truncated-conical portion of the rotating basket (2), corresponding to the beginning of the squeezing section (3).

6. The device according to claim 1, wherein the angle α ranges from 9 to 13°.

7. The device according to claim 1, wherein the S'/C' ratio ranges 0.5 to 0.65.

8. The device according to claim 3, wherein a reduction ratio between the rotating basket and the screw ranges from 95:1 to 70:1.

9. The device according to claim 8, wherein the reduction ratio ranges from 90:1 to 77:1.

## Patentansprüche

1. Vorrichtung zur Implementierung eines Verfahrens zum Abtrennen von Melaminkristallen in einer Anlage zur Synthese von Melamin aus Harnstoff, wobei die Vorrichtung eine Vollkorbzentrifuge ist, die aus einem stationären Außengehäuse (1) besteht, in dem ein rotierender Korb (2) positioniert ist, der zur Trennung der beiden Phasen, fest und flüssig, geeignet ist, wobei der rotierende Korb (2) aus einem hohlen zylindrischen Körper C besteht, der an der Auslassseite des feuchten Kuchens mit einem Quetschabschnitt S (3) aufweisend eine kegelstumpfförmige Form endet, wobei der kegelstumpfförmige Quetschabschnitt S (3) durch einen Winkel a im Bereich von 6 bis 16° gekennzeichnet ist, wobei, gegenüber der Rotationsebene gemessen, der Winkel a der Neigungswinkel des Abschnitts S ist und die Vorrichtung **dadurch gekennzeichnet ist, dass** das S'/C'-Verhältnis im Bereich von 0,45 bis 0,85 liegt, wobei C' die Länge des zylindrischen Körpers und S' die Projektionslänge des kegelstumpfförmigen Quetschabschnitts (S) auf der Drehachse ist und die Vorrichtung sich in einer Anlage für die Synthese von Melamin aus Harnstoff befindet.

2. Vorrichtung nach Anspruch 1, **dadurch gekennzeichnet, dass** die Zentrifuge eine Extraktionsschraube (6) umfasst, deren Differentialbewegung gegenüber dem rotierenden Korb (2) durch das Reduzierstück (7) sichergestellt ist.

3. Vorrichtung nach Anspruch 2, **dadurch gekennzeichnet, dass** die Extraktionsschraube (6) eine Doppelspiralschraube ist.

4. Vorrichtung nach einem oder mehreren der Ansprüche 1-3, **dadurch gekennzeichnet, dass** der 0 rotierende Korb (2) einstellbare Öffnungen (8) aufweist, deren Position die Höhe der flüssigen Phase innerhalb des rotierenden Korbs (2) und folglich die von der flüssigen Phase nicht abgedeckten Nutzlänge des Quetschabschnitts (3), mit dem gleichen Winkel a und S'/C'-Verhältnis zwischen der Länge des zylindrischen Abschnitts und der Projektionslänge des kegelstumpfförmigen Abschnitts auf der Rotationsachse des rotierenden Korbs bestimmt.

5. Vorrichtung nach einem oder mehreren der Ansprüche 1-4, **dadurch gekennzeichnet, dass** die Position (10) des Einlasspunkts (4) der zugeführten Aufschlämmung dem Verbindungspunkt zwischen dem zylindrischen Abschnitt und dem kegelstumpfförmigen Abschnitt des rotierenden Korbs (2), entsprechend dem Beginn des Quetschabschnitts (3) entspricht.

6. Vorrichtung nach Anspruch 1, wobei der Winkel a im Bereich von 9 bis 13° liegt.

7. Vorrichtung nach Anspruch 1, wobei das S'/C'-Verhältnis im Bereich von 0,5 bis 0,65 liegt.

8. Vorrichtung nach Anspruch 3, wobei ein Reduktionsverhältnis zwischen dem rotierenden Korb und der Schraube im Bereich von 95:1 bis 70:1 liegt.

9. Vorrichtung nach Anspruch 8, wobei das Reduktionsverhältnis im Bereich von 90:1 bis 77:1 liegt.

## Revendications

1. Dispositif pour mettre en œuvre un procédé pour la séparation de cristaux de mélamine dans une installation pour la synthèse de mélamine à partir d'urée, ledit dispositif étant une centrifugeuse à bol plein, constituée d'un boîtier extérieur (1) stationnaire dans lequel un bol rotatif (2) est positionné, adaptée pour séparer les deux phases, solide et liquide, ledit bol rotatif (2) constitué d'un corps C cylindrique creux terminant, sur le côté de sortie du gâteau humide, par une section de resserrement S (3) ayant une forme tronconique, ladite section de resserrement S (3) tronconique étant **caractérisée par** un angle α allant de 6 à 16°, ledit angle α étant l'angle d'inclinaison de ladite section S, mesuré par rapport au plan de rotation et ledit dispositif étant **caractérisé en ce que** le rapport S'/C' va de 0,45 à 0,85, C' étant la longueur du corps cylindrique et S' étant la longueur de projection de la section de resserrement (S) tronconique sur l'axe de rotation ; ledit dispositif étant dans une installation pour la synthèse de mélamine à partir d'urée.

2. Dispositif selon la revendication 1, **caractérisé en ce que** la centrifugeuse comprend une vis d'extraction (6), dont le mouvement différentiel par rapport au bol rotatif (2) est assuré par le réducteur (7).

3. Dispositif selon la revendication 2, **caractérisé en ce que** la vis d'extraction (6) est une vis à double spirale.

4. Dispositif selon l'une ou plusieurs des revendications 1-3, **caractérisé en ce que** le bol rotatif (2) a des ouvertures (8) ajustables, dont la position détermine la hauteur de la phase liquide à l'intérieur du bol rotatif (2) et par conséquent la longueur utile, non couverte par la phase liquide, de la section de resserrement (3), avec le même angle α et rapport S'/C' entre la longueur de la portion cylindrique et la longueur de projection de la portion tronconique sur l'axe de rotation du bol rotatif.

5. Dispositif selon l'une ou plusieurs des revendications 1-4, **caractérisé en ce que** la position (10) du point d'entrée (4) de la suspension alimentée correspond au point de raccordement entre la portion cylindrique et la portion tronconique du bol rotatif (2), correspondant au début de la section de resserrement (3).

6. Dispositif selon la revendication 1, dans lequel l'angle α va de 9 à 13°.

7. Dispositif selon la revendication 1, dans lequel le rapport S'/C' va de 0,5 à 0,65.

8. Dispositif selon la revendication 3, dans lequel un rapport de réduction entre le bol rotatif et la vis va de 95:1 à 70:1.

9. Dispositif selon la revendication 8, dans lequel le rapport de réduction va de 90:1 à 77:1.
